# EUROPEAN PATENT SPECIFICATION

(11) **EP 2 376 644 B1**
(45) Date of publication and mention of the grant of the patent: **23.04.2014**
(21) Application number: 10700202.4
(22) Date of filing: 11.01.2010
(51) Int. Cl.: C12P 17/18, C12P 19/42, C12P 39/00, C12N 1/20, A61K 35/74, A23L 1/302

(54) **Process for the preparation of a fermentation broth**
Verfahren zur Herstellung einer Fermentationsbrühe
Procédé de préparation d'un moût de fermentation

(30) Priority: 12.01.2009 CH 31092009
(43) Date of publication of application: 19.10.2011
(60) Divisional application: 13004233.6
(73) Proprietor: Bioforce AG Roggwil TG, 9325 Roggwil (CH)
(72) Inventor: HUGENSCHMIDT, Selina, CH-4310 Rheinfelden (CH); MIESCHER SCHWENNINGER, Susanne, LI-9494 Schaan (LI); LACROIX, Christophe, CH-8802 Kilchberg (CH)
(74) Representative: Zink-Wild, Markus Peter
(86) International application number: PCT/CH2010/000006
(87) International publication number: WO 2010/078670

(56) References cited:
- CERNA J ET AL: "BIOLOGIC ENRICHMENT OF FERMENTED MILK BEVERAGES WITH VITAMIN B12 AND FOLIC ACID" MILCHWISSENSCHAFT, VV GMBH VOLKSWIRTSCHAFTLICHER VERLAG. MUNCHEN, DE, vol. 32, no. 5, 1 January 1977 (1977-01-01), pages 274-277, XP008044088 ISSN: 0026-3788
- DATABASE WPI Week 200518 Thomson Scientific, London, GB; AN 2005-170041 XP002593873 & RU 2 243 678 C1 (BIOTEKH ENGG STOCK CO) 10 January 2005 (2005-01-10)
- DATABASE WPI Week 198705 Thomson Scientific, London, GB; AN 1987-036279 XP002593874 & SU 1 238 746 A1 (DAIRY IND RES INST) 23 June 1986 (1986-06-23)
- BERRY E C ET AL: "USE OF CHEESE WHEY FOR VITAMIN B12 PRODUCTION II. COBALT, PRECURSOR, AND AERATION LEVELS" APPLIED MICROBIOLOGY, AMERICAN SOCIETY FOR MICROBIOLOGY, US, vol. 14, no. 3, 1 May 1966 (1966-05-01), page 356/357, XP009008814 ISSN: 0003-6919 cited in the application
- SYBESMA WILBERT ET AL: "Effects of cultivation conditions on folate production by lactic acid bacteria." APPLIED AND ENVIRONMENTAL MICROBIOLOGY, vol. 69, no. 8, August 2003 (2003-08), pages 4542-4548, XP002593875 ISSN: 0099-2240 cited in the application
- MARTENS J -H ET AL: "Microbial production of vitamin B12" APPLIED MICROBIOLOGY AND BIOTECHNOLOGY, vol. 58, no. 3, March 2002 (2002-03), pages 275-285, XP002593876 ISSN: 0175-7598 cited in the application
- WINKELS RENATE M ET AL: "Bread cofortified with folic acid and vitamin B-12 improves the folate and vitamin B-12 status of healthy older people: a randomized controlled trial" AMERICAN JOURNAL OF CLINICAL NUTRITION, vol. 88, no. 2, August 2008 (2008-08), pages 348-355, XP002593877 ISSN: 0002-9165 cited in the application
- HUGENHOLTZ JEROEN ET AL: "Nutraceutical production with food-grade microorganisms" CURRENT OPINION IN BIOTECHNOLOGY, vol. 13, no. 5, October 2002 (2002-10), pages 497-507, XP002593878 ISSN: 0958-1669 cited in the application
- GONZALEZ SISO M I: "The biotechnological utilization of cheese whey: A review" BIORESOURCE TECHNOLOGY, vol. 57, no. 1, 1996, pages 1-11, XP002593879 ISSN: 0960-8524 cited in the application
- LEBLANC J G ET AL: "Folate production by lactic acid bacteria and other food-grade microorganisms", 1 January 2007 (2007-01-01), COMMUNICATING CURRENT RESEARCH AND TRENDS IN APPLIED MICROBIOLOGY, FORMATEX, EXTREMADURA - SPAIN, PAGE(S) 329 - 339, XP002547461, ISBN: 978-84-611-9422-3

## Description

The present invention is directed to a process for the preparation of a fermentation broth.

Whey is a by-product of the cheese industry and still contains about 55 % of total milk nutrients with 44 to 52 g/l lactose, 6 to 10 g/l proteins, 3 to 7 g/l minerals, and about 4 to 5 g/l fat. There are two types of whey: (1) sweet whey from rennet-induced coagulation and (2) acid whey from fermentations or from acid-coagulation. Whey permeate is obtained after ultrafiltration of whey and contains less than 1.5 g/l protein. Disposal of whey and whey permeate leads to significant waste of potential food and energy and to severe environmental problems; it affects the physical and chemical structure of soil and reduces the aquatic life by depleting the dissolved oxygen (Panesar *et al.,* 2007; Vasala *et al.,* 2005; Gonzales Siso, 1996).

Whey utilization has been the subject of extensive research and a wide range of products can be obtained from whey and whey fermentations, such as lactose, ethanol, microbial biomass, organic acids, and fermented beverages. However, recovery costs dominate the economics of the processes (Mawson, 2003; Gonzales Siso, 1996). Furthermore, supplementation with easy digestible N-compounds is mandatory for using whey as a substrate for fermentations (Lund *et al.,* 1992).

Lactic acid bacteria, abbreviated with LAB, and propionic acid bacteria, abbreviated with PAB, have a long tradition in food fermentations. LAB cause rapid acidification through the production of organic acids and produce other substances, such as aroma compounds, bacteriocins and exopolysaccharides. Some LAB are known to produce folate (Leroy and De Vuyst, 2004).

The term "folate" is a non-specific term referring to any folate compound with vitamin activity and a basic structure similar to folic acid. The term "folic acid" is used for the chemically synthesized vitamin (Hugenholtz and Smid, 2002).

In the present invention the term "folate" is used to describe at least one folate compound with vitamin activity.

PAB produce propionate and acetate as main fermentation end products, but are also known for the production of vitamin B12 in large amounts as well as folate (Hugenholtz *et al.,* 2002; Hettinga and Reinbold, 1972).

The term "vitamin B12" is used to describe any compound of the cobalamin group. Cyanocobalamin, vitamin B12 by definition, is produced industrially but is not found in nature (Piao *et al.,* 2004).

In the present invention the term "vitamin B12" is used to describe at least one compound of the cobalamin group, for example hydroxocobalamin, aquoco-balamin, nitritocobalamin, adenosylcobalamin, and me-thylcobalamin.

Folate biosynthesis starts from the 3 precursors guanosine triphosphate, abbreviated with GTP, p-aminobenzoate, abbreviated with pABA, and glutamate and is produced by many plants, some fungi and bacteria (Quinlivan *et al.,* 2006; Sybesma *et al.,* 2003).

Vitamin B12 is only produced by bacteria. Its biosynthesis is different in aerobic and anaerobic microorganisms and starts from the 3 precursors uroporphy-rinogen III, 5,6-dimethylbenzimidazole, abbreviated with DMBI, and an adenosyl-moiety in the case of adenosylcobalamin (Hugenholtz and Smid, 2002; Martens *et al.,* 2002).

In PAB, production is divided in 2 stages: an anaerobic stage for the first 3 days of fermentation, where the bacteria produce the vitamin B12 precursor cobamide, followed by an aerobic stage for 1 to 3 days for the synthesis of DMBI and its linkage to cobamide. The exact explanation for the aerobic stimulation of vitamin B12 production, however, is not yet known (Murooka *et al.,* 2005; Hugenholtz *et al.,* 2002).

Nevertheless, for an improved vitamin B12 production, the addition of cobalt ions and DMBI in a 2-step fermentation process led to major improvement in vitamin B12 production in all cases (Survase *et al.,* 2006).

The time of addition of DMBI thereby seems to be important and should occur during the last 24 h of fermentation, since Marwaha *et al.* (1983) found that DMBI added in an earlier stage inhibited growth and vitamin B12 production of PAB. Similar, Berry and Bullerman (1966) reported on negative effects of DMBI on vitamin B12 production if given at the beginning of fermentation compared to no addition of DMBI.

LAB and PAB often grow in mixed cultures, such as in dairy products (e.g. Swiss cheese) or in silage. Interactions of mixed cultures can have a positive, neutral or negative effect on the fitness of the cells, depending on species and strains (Sieuwerts *et al.,* 2008; Liu and Moon, 1982).

Furthermore, there are LAB - PAB co-cultures described with increased antimicrobial properties if grown together compared to individual cultures (Miescher Schwenninger and Meile, 2004).

Organic acids produced by these bacteria, i.e. acetate and propionate, are strong antimicrobial agents with minimal inhibitory concentrations (MIC) of 50 to 500 mM for acetate and 10 to 670 mM for propionate, depending on pH and organism (Miescher Schwenninger *et al.,* 2008; Suomalainen and Mayra-Makinen, 1999).

Sufficient intake of folate in industrialized countries is assumed to be critical due to low folate concentrations in the daily food. Average intakes are estimated to be 200 to 300 µg per day, and the adult recommended dietary allowance (RDA) for folate is set to 200 to 400 µg per day (Tönz, 2002; Forssén *et al.,* 2000).

Contrary, vitamin B12 intake is above the RDA of 1 to 3 µg per day and deficiencies may occur only in strict vegans and people having gastro-intestinal or other disorders. However, due to a reduced absorption, vitamin B12 deficiency of more than 20% in elderly people is recognized (Ryan-Harshmann and Aldoori, 2008; Frank, 2002).

Folates are biologically active by carrying C-1 units for the production of DNA and RNA but also for the metabolism of certain amino acids. A low folate status leads to megaloblastic anemia, an increased incidence of birth defects, is a risk factor for cancer and coronary vascular diseases, and is linked to dementia and Alzheimer (Bekaert *et al.,* 2008; Witthöft and Jager-stad, 2002).

Folate stands in close metabolic relationship to vitamin B12 via methionine transferase, which is a vitamin B12 dependent enzyme needed to restore folate back to its active form (Reynolds, 2006).

As for folate, vitamin B12 deficiencies lead to megaloblastic anemia, but also to neurological and psychiatric disorders (Truswell, 2007).

A major concern of a folate supplementation in humans is the masking of a vitamin B12 deficiency and therefore an irreversible damage to the nervous system (Stover, 2004).

Several other potential negative impacts have been reported for folate supplementation in the presence of a low vitamin B12 status. New evidence suggested that persons with a low vitamin B12 status and high folate concentrations were at high risk of memory impairment and anemia (Refsum and Smith, 2008; Johnson, 2007).

Recent data thus indicate the importance to maintain a good balance between folate and vitamin B12. Co-fortification with folate and vitamin B12 could simultaneously improve folate and vitamin B12 status (Winkels *et al.,* 2008). Based on dietary recommendations, a folate to vitamin B12 ratio of 130 - 200:1 would be optimal for food supplementation.

Recent data also suggested the need to distinguish naturally occurring folate from the synthetic form of folate, called folic acid, which is produced commercially by chemical synthesis and added to supplements and fortified foods (Wright *et al.,* 2007; Ulrich and Potter, 2006; Lucock, 2000). It was shown that the absorption and biotransformation of synthetic folic acid has a saturation level, which, if exceeded, might raise the possibility of an exposure to unmetabolized folic acid. The health risk of such an exposure is not yet known in detail, but it might increase the risk for some cancers.

Synthetic folic acid is cheap to produce and highly stable in contrast to most of the natural forms of this vitamin. Several methods for synthesizing folic acid have been described, but so far only yields of 12.6 -84% were obtained depending on the process (Wehrli, 1995). In 1991, ca. 300 t of folic acid were produced worldwide with a value of about 30 million USD (Eurolex, 2003).

In contrast to the chemically synthesized folic acid, vitamin B12 is exclusively produced by biosynthetic fermentation processes, because a chemical synthesis is far too technically challenging and expensive. More than 10 t of vitamin B12 are produced each year. High vitamin B12 yields are thereby obtained using selected and genetically optimized microorganisms (Martens *et al.,* 2002).

Using food grade microorganisms, however, has the advantage that vitamins can be produced *in situ* in food, which makes a complex extraction and purification of the vitamins unnecessary.

Furthermore, products containing adequate concentrations of naturally produced folate and vitamin B12 can avoid the problematic intake of synthetic folic acid since no adverse health risks of naturally produced folate are known. At the same time they may contribute to a sufficient and balanced intake of both vitamins which is important for their beneficial effects.

Biological enrichment of fermented milk beverages with vitamin B12 and folic acid was achieved by the addition of a specific Propionibacterium shermanii strain as described by Cerna and Hrabova (1977).

Furthermore, a method for preparing a protein-vitamin fodder involving an incubation of pairs of specific Lactobacillus acidophilus strains with specific Propionibacterium acnes or P. freudenreichii subsp. shermanii strains in processed biological waste products was described by Galkina et al. (2003).

It is an object of the present invention to reduce the year-to-year increasing amount of whey that is obtained as a by-product during cheese production.

Whey shall be transformed into a convertible and demanded new product that shall have a high market value.

It is a further object of the present invention to provide a process for the preparation of a fermentation broth.

In this process whey shall be used as growth medium, especially supplemented whey and/or supplemented whey permeate, whereby said whey is preferably obtained as a by-product during cheese production.

The fermentation broth obtained by this process shall contain naturally produced folate and naturally produced vitamin B12 in adequate concentrations.

The fermentation broth obtained by this process shall have a nutritional value and/or antimicrobial properties.

In this process bacteria shall be used that produce folate and/or vitamin B12.

This process shall be simple and cost advantageous.

With the present invention these objects are achieved.

Quite surprisingly it has been found that applying co-cultures consisting of folate and vitamin B12 producing LAB and PAB strains and using their complementary metabolisms for a simultaneous production of both vitamins, can be used to produce such a fermentation broth.

In addition, using co-cultures of folate and vitamin B12 producing LAB and PAB strains leads to an increased folate content compared to a single culture with only LAB.

The present invention is directed to a process for the preparation of a fermentation broth.

This process is characterized in that a mixture of
- *Lactobacillus plantarum* SM39,
- *Propionibacterium freudenreichii* DF13 and
- supplemented whey permeate, whereby said whey permeate is preferably obtained either from ultrafiltration of milk or whey, whereby said whey is a by-product from cheese production, and whereby said supplemented whey permeate contains added cobalt chloride in an amount of 5 mg/l,
   whereby said supplementation comprises the addition of
- 0.02 % (w/v) magnesium sulfate,
- 0.005 % (w/v) manganese sulfate,
- 1 % (w/v) yeast extract,
- p-amino benzoic acid in an amount of 10 mg/l,
- 5,6-dimethylbenzimidazole in an amount of 15 mg/l and
- a buffer consisting of a 0.1 M aqueous solution of K₂HPO₄ and KH₂PO₄ having a pH value of 6.4,
whereby 10⁵ cfu/ml of said *Lactobacillus plantarum* SM39 and 10⁷ cfu/ml of said *Propionibacterium freudenreichii* DF13 are present at the beginning of the cultivation,
is prepared, then
this mixture is cultivated for 72 hours under anaerobic conditions under a N₂ atmosphere, followed by a cultivation under aerobic conditions during at least 24 hours, preferably during 72 hours,
whereby said cultivation is carried under stirring at a temperature of 33°C at a pH value of 6.1 and whereby said pH value is controlled by the addition of 5 M NaOH, and
finally the resulting fermentation broth is collected.

Preferred embodiments of this invention are defined in the dependent claims.

In the following part possible embodiments of the present invention are described.

The following examples shall illustrate the present invention.

### Example 1 (Preparation of a fermentation broth)

All chemicals were obtained from Sigma-Aldrich Chemie GmbH, Buchs, Switzerland, unless otherwise stated.

### A. Inoculum

*Lactobacillus plantarum* SM39 / DSM 22118 - deposited on 15.12. 2008 at DSMZ (Braunschweig, Germany) - and *Propionibacterium freudenreichii* DF13 / DSM 22120 - deposited on 15.12. 2008 at DSMZ (Braunschweig, Germany) - were kept separately as frozen stocks at a temperature of -80°C in glycerol (1:1) and subcultured separately at least three times at a temperature of 30°C in supplemented whey permeate (abbreviated with SWP; see below) prior to the preparation of the inoculum. OD600 (optical density, measured at 600 nm) of freshly grown cultures (4 d for PAB, 1 d for LAB) was measured and set to 2.0 for PAB and 0.2 for LAB, corresponding to 10⁹ and 10⁷ cfu/ml [colony forming unit/ml], respectively. A 1-% inoculum of each of these standardized cultures was then used to inoculate the fermentors.

### B. Preparation of supplemented whey permeate (SWP)

SWP consisted of 6 % whey permeate (w/v), 0.98 % K₂HPO₄ (w/v), 0.48 % KH₂PO₄ (w/v), 0.02 % magnesium sulfate (w/v), 0.005 % manganese sulfate (w/v), and 1 % yeast extract (w/v) and had a final pH of 6.2. Its preparation was done as follows:

8.6 % whey permeate (w/v; Emmi Schweiz AG, Dagmersellen, Switzerland, "Permeatpulver" article number 1000074/FP1076) with pH adjusted to 5.0 (5 M HCl) was autoclaved (121°C, 15 min) and filtered (cellulose acetate membrane, 0.2 µm; VWR International AG, Dietikon, Switzerland). 700 ml of this autoclaved and filtered whey permeate was mixed with 150 ml of a Mg/Mn solution [2.73 g/l magnesium sulfate heptahydrate and 0.37 g/l manganese sulfate monohydrate] and autoclaved again (121°C, 15 min).

Additionally, 100 ml 1 M potassium phosphate buffer (pH 6.4) and 50 ml 20 % yeast extract (w/v, VWR International AG, Dietikon, Switzerland) were mixed and autoclaved (121°C, 15 min).

Said 1 M potassium phosphate buffer was prepared by mixing 27.8 ml of 1 M K₂HPO₄ and 72.2 ml of 1 M KH₂PO₄.

Finally, both solutions were either aseptically mixed in a sterile flask for the preparation of the inoculum or aseptically added to the fermentor with the aid of a sterile funnel together with the standardized inoculum.

For efficient growth of PAB for the preparation of the inoculum, lactate was added to SWP. Therefore, 1.3 % sodium D/L- lactate syrup 60 % (w/v), 50 ml 20 % yeast extract (w/v), and 100 ml 1 M potassium phosphate buffer (pH 6.6) were mixed and autoclaved (121°C, 15 min). Phosphate buffer with a pH of 6.6 (instead of 6.4) was chosen in order to get the same final pH of 6.2 after autoclaving, as for SWP without addition of lactate.

Said 1 M potassium phosphate buffer was prepared by mixing 38.1 ml of 1 M K₂HPO₄ and 61.9 ml of 1 M KH₂PO₄.

### C. Fermentation conditions

7-days batch fermentations were carried out in 1-liter bioreactors (Multiforce, Inforce HT, Bottmingen, Switzerland) under anaerobic conditions for 3 days, followed by aerobic conditions for 4 days. Fermentors were equipped with an aseptic sampling device, temperature control, pH electrode, base pump, air and base inlet. Fermentation parameters are shown in Table 1.

**Table 1: Fermentation parameters.**

| Parameter | Conditions |
|---|---|
| Medium | SWP + 5 ppm cobalt chloride + 10 ppm pABA + 15 ppm DMBI (the latter added for the last 2 days of fermentation) |
| Inoculum | 1 % of 10⁷ cfu/ml *L. plantarum* SM39 / 1 % of 10⁹ cfu/ml *P. freudenreichii* DF13 |
| Volume | 500 ml |
| Temperature | 33°C |
| Stirring | 150 rpm |
| pH | controlled at 6.1 by addition of 5M NaOH |
| Atmosphere | 3 days anaerobic followed by 4 days aerobic |

Anaerobic conditions were obtained by a continuous flow of pure N₂ (headspace, 1.2 bar, 0.15 l/min), containing not more than 3 ppm of O₂, and aerobic conditions by a continuous flow of air (headspace, 1.2 bar, 0.1 l/min). 10 ml of sterile filtrated (0.2-µm filter; Sartorius Minisart-Plus; VWR International AG, Dietikon, Switzerland) DMBI (0.75 mg/ml) were added for the last 2 days of fermentation. 5 ml of sterile filtrated pABA (1 mg/ml) and 0.5 ml of cobalt chloride (5 mg/ml) were added at the beginning of fermentation.

At the end of fermentation the resulting cultured medium was collected.

During fermentation, cell counts, organic acids, sugars and vitamins (folate and vitamin B12) were controlled regularly. Therefore, 5 to 25 ml samples were collected every 3 h on the first day (day 0) and once a day from day 1 to 7 for the following analyses: plate counts, HPLC analyses for sugars and organic acids, microbiological determination of folate, HPLC analyses for vitamin B12 (day 3 to 7), and microscopic analysis. In addition, a 40-ml sample collected on day 7 was stored at a temperature of -20°C. Plating as well as folate and vitamin B12 analyses were done immediately after collecting the samples. For HPLC analyses of sugars and organic acids, the samples were centrifuged (10'000 x g, 10 min; Centrifuge 5417R, Eppendorf, Germany), sterile filtrated (0.2-µm filter; Sartorius Minisart-Plus; VWR International AG, Dietikon, Switzerland) and the cell-free supernatants were stored at a temperature of -20°C for a maximum of 2 month before analysis.

### D. Bacterial enumeration by plate count:

Samples were serially diluted in NaCl-peptone water (0.85% NaCl (w/v; Mallinckrodt Baker, Inc., USA) - 0.1% peptone (w/v)) and drops of 20 µl of appropriate dilutions were placed on agar plates. For enumeration of LAB, "MRS medium" (Labo-Life Sàrl, Pully, Switzerland; De Man *et al.,* 1960) was used and for PAB NL medium (sodium lactate medium), containing 1 % tryptic soy broth without dextrose (w/v; Beckton Dickinson, Allschwil, Switzerland), 1 % yeast extract (w/v; VWR International AG, Dietikon, Switzerland), and 1.3 % sodium D/L-lactate syrup 60 % (w/v) according to Grinstead and Barefoot (1992). After drying the drops, the plates were incubated anaerobically at a temperature of 30°C for 6 days for PAB and at a temperature of 37°C for 2 days for LAB. On NL medium only brown colonies were counted that corresponded to PAB. Cell counts were performed in duplicate and mean values were expressed as Log10 cfu/ml fermented medium.

### E. Folate (microbiological assay) analysis:

Extracellular folate were quantified using a microbiological assay with *L. casei* ATCC7469 as indicator strain in microtiter plates according to Horne and Patterson (1988), with the following modifications:

### E1. Preparation of standardized inoculum:

The inoculum of the indicator strain *L. casei* ATCC7469 was prepared according to Becton Dickinson, modified as follows: 10 ml "Micro Inoculum Broth" (Beckton Dickinson, Allschwil, Switzerland) were inoculated with 1 % *L. casei* ATCC7469 and incubated at a temperature of 37°C over night. This procedure was repeated once again. The second culture was centrifuged (10'000 x g, 10 min, 4°C; Centrifuge 5417R, Eppendorf) and the pellet was washed 4-times with a 0.85-% NaCl solution (Mallinckrodt Baker, Inc., USA). After washing, the pellet was resuspended in 10 ml 2-fold concentrated "Folic Acid Casei Medium" (Beckton Dickinson, Allschwil, Switzerland) and 100-fold diluted with fresh 2-fold concentrated "Folic Acid Casei Medium". Volumes of 1 ml were filled into "cryo vials" (Huber + Co, Reinach BL, Switzerland) containing 1 ml 80 % sterile glycerol and were stored at a temperature of -80°C until usage.

### E2. Preparation of samples:

2-ml fermentation samples - as obtained in the above step C - were centrifuged (10'000 x g, 10 min; Centrifuge 5417R, Eppendorf) and sterile filtrated (0.2-µm filter; Sartorius Minisart-Plus; VWR International AG, Dietikon, Switzerland). They were 1'500 - to 15'000-fold diluted in folate buffer [0.1 M sodium phosphate buffer, pH 6.8, 0.1 % ascorbic acid (VWR International AG, Dietikon, Switzerland), 0.01 M 2-mercaptoethanol] in order to obtain folate concentrations in the range from 0.0312 to 0.625 ng/ml and were stored at a temperature of 4°C in dark for a maximum of 4 days before performing the assay.

### E3. Assay:

Each microtiter plate (Optical 96-Well Reaction Plate with Barcode; Applied Biosystems, Rotkreuz, Switzerland) was filled with either 150 µl sample - as obtained in the above step E2 - or with folic acid standard (Schircks Laboratories, Rapperswil-Jona, Switzerland). This folic acid standard was prepared in folate buffer [0.1 M sodium phosphate buffer pH 6.8, 0.1 % ascorbic acid (VWR International AG, Dietikon, Switzerland), 0.01 M 2-mercaptoethanol] in a range from 0.0078 to 10 ng/ml. Two "cryo vials" with standardized inoculum - as obtained in the above step E1 - were thawed and added to 10 ml 2-fold concentrated "Folic Acid Casei Medium". 150 µl of this diluted inoculum were then added to the above mentioned 150 µl samples or standards resulting in a final volume of 300 µl per well. Growth of the indicator organism *L*. *casei* ATCC7469 was determined by measuring optical density at 600 nm before and after an anaerobic incubation period of 20 h at a temperature of 37°C. Folate concentrations of the samples were calculated by comparing the optical density of said samples and the standard solutions and were expressed in ng/ml.

### F. Vitamin B12 (HPLC) analysis:

Vitamin B12 was analyzed according to Piao et a1. (2004) with some modifications as described in the following steps F1 and F2. Vitamin B12 determination was done with HPLC after its extraction from the cells and its conversion into cyanocobalamin.

### F1. Sample preparation:

20 ml fermentation sample - as obtained in the above step C - were centrifuged (10'000 x g, 15 min; Biofuge Primo Hexaeus) and the pellet was washed with a 0.2 M potassium phosphate buffer (pH 5.5), centrifuged, and resuspended in 1 ml of 0.2 M potassium phosphate buffer (pH 5.5) containing 0.1 % potassium cyanide. The samples were "vortexed" (shaked) and autoclaved (121°C, 15 min). After autoclaving, they were "vortexed" again and centrifuged (10'000 x g, 15 min; Biofuge Primo Heraeus). The supernatant was filtrated (0.45-µm nylon membrane filters; Infochroma, Zug, Switzerland) into HPLC vials (Infochroma, Zug, Switzerland), sealed with aluminum crimp caps (Infochroma, Zug, Switzerland), and stored at a temperature of 4°C in dark for a maximum of 4 days before HPLC analysis.

### F2. HPLC:

HPLC was performed on a reversed-phase C18 column (250x4.6 mm; 5 µm particle size; Atlantis; Waters, US) with a RP C18 guard cartridge (20x4.6 mm; 5 µm particle size; Atlantis; Waters, US) on a HPLC system containing a Merck Hitachi L-7100 pump, a Merck Hitachi L-7200 autosampler with a Peltier sample cooler, a VWR Scientific Instruments column oven II plus, a Merck Hitachi L-7455 DAD Detector, and a Merck Hitachi D-7000 HPLC system manager. An acetonitrile / MilliQ water gradient mobile phase (Table 2) was used and UV detection was done at 358 nm. Flow rate was set to 1.4 ml/min, oven temperature to 30°C, and injection volume was 40 µl. Cyanocobalamin standards in the range of 1 to 20 µg/ml in MilliQ water were used. Each sample was injected twice and means were calculated. With this method, the detection limit for vitamin B12 was set at 30 ng/ml fermented medium.

**Table 2: Time dependent gradient of the eluents MilliQ water and acetonitrile for vitamin B12 determination using HPLC.**

| Time [min] | MilliQ water [%] | acetonitrile [%] |
|---|---|---|
| 0 | 100 | 0 |
| 5 | 95 | 5 |
| 7 | 85 | 15 |
| 14 | 80 | 20 |
| 17 | 0 | 100 |
| 20 | 100 | 0 |
| 25 | 100 | 0 |

### G. HPLC analyses of organic acids and sugars:

Cell-free supernatants of the fermentation samples - as obtained in the above step C - were thawed and 10-fold diluted in MilliQ water (Millipore AG, Zug, Switzerland). The samples were directly filtrated into HPLC vials (Infochroma, Zug, Switzerland) using 0.45-µm nylon membrane filters (Infochroma, Zug, Switzerland), sealed with aluminum crimp caps (Infochroma,-Zug, Swit-zerland), and analyzed with HPLC.

HPLC was performed on a HPX-87H column (300x7.8 mm; Animex; BioRad, Switzerland) with a Cation-H guard cartridge (30x4.6 mm; BioRad, Switzerland) on a HPLC system consisting of a Merck Hitachi L-7100 pump, a Merck Hitachi L-7200 autosampler with a Peltier sample cooler, a Merck Hitachi L-7360 column oven, a Merck Hitachi L-7450 DAD detector and a L-2490 RI detector, and a Merck Hitachi D-7000 HPLC system manager. As eluent 10 mM H₂SO₄ in MilliQ water was used. Flow rate was set to 0.6 ml/min, oven temperature to 40°C, and injection volume was 40 µl. Standards were prepared in MilliQ water in the range of 0.24 to 6 g/l for lactic acid, 0.04 to 1.0 g/l for propionic acid, acetic acid, glucose and galactose, and 0.2 to 5 g/l for lactose. Each sample was injected twice and mean values were calculated. With this method, the detection limits for the different substances in water were 0.18 g/l lactic acid, 0.03 g/l for each propionic acid, acetic acid, glucose and galactose, and 0.13 g/l lactose.

### H. Results

Folate yields, vitamin B12 yields, and cell counts obtained during a fermentation of *L. plantarum* SM39 and *P. freudenreichii* DF13 under anaerobic conditions for 3 days, followed by aerobic conditions for 4 days in SWP with 5 ppm cobalt chloride, 15 ppm DMBI, and 10 ppm pABA - as described in the above step C - are shown in Table 3.

Folate concentrations continuously increased from the start of fermentation to day 3. From day 3 to day 7 the daily folate concentration remained stable at an average level-of 6317 ± 1281 ng/ml (Table 3). Concerning vitamin B12, a concentration of 869 ± 524 ng/ml was reached on day 5. After addition of DMBI on day 5, vitamin B12 concentrations increased to a mean value of 1065 ± 378 ng/ml for days 6 and 7. Cell numbers increased from initially 5 and 7 Log cfu/ml for *L. plantarum* SM39 and *P. freudenreichii* DF13, respectively, to about 9.5 Log cfu/ml after 2 days of fermentation for both strains and remained stable thereafter with a balanced ratio. Lactose (46 ± 3 g/l at the beginning) was completely metabolized after 3 days, concomitant with an increase in lactate. Lactate concentration reached a maximum after 2 days (5 ± 1 g/l) and decreased afterwards due to consumption by *Propionibacterium* concomitant with an increase in acetate and propionate to reach 9 ± 0.1 g/l and 21 ± 2 g/l, respectively, on day 4.

**Table 3: Cell counts, extracellular folate, and intracellular vitamin B12 production (Mean values and standard deviation of double determinations).**

| Cell counts [Log cfu/ml] | | | | Folate [ng/ml] | Vitamin B12 [ng/ml] |
|---|---|---|---|---|---|
| LAB | | PAB | | | |
| 0 h | d2 - d7^{a,b} | 0 h | d2 - d7^{a,b} | d3-d7^{a,c} | d6-d7^{a,d} |
| 4.97 | 9.55 | 7.06 | 9.53 | 6317 | 1065 |
| ± 0.13 | ± 0.03 | ± 0.01 | ± 0.02 | ± 1281 | ± 378 |

| | | | | | |
|---|---|---|---|---|---|
| a) average values over several days were calculated for constant cell growth / vitamin production to show the stability. b) average daily cell counts from day 2 to 7. c) average daily folate concentrations from day 3 to 7. d) average daily vitamin B12 concentrations from day 6 and 7. | | | | | |

### Example 2 (Preparation of a fermentation broth)

The same fermentation as described in Example 1 was repeated, with the exception that as inoculum a single culture of 1 % of 10⁷ cfu/ml *L. plantarum* SM39 was used instead of the co-culture.

### Results of Example 2

Folate concentrations continuously increased from the start of fermentation to day 3. From day 3 to day 7 the daily folate concentration remained stable at an average level of 4293 ± 193 ng/ml (Table 4). Cell numbers increased from initially 5 Log cfu/ml for L. plantarum SM39 to about 9.6 Log cfu/ml after 3 days of fermentation and remained stable thereafter. Lactose (43 ± 1 g/l at the beginning) was completely metabolized after 4 days, concomitant with an increase in lactate. Lactate concentration reached a maximum after 3 days (36 ± 4 g/l), remained stable for 3 days and decreased afterwards slightly. Acetate continuously increased to a concentration of 2.4 ± 0.01 g/l on day 7.

**Table 4: Cell counts and extracellular folate production (Mean values and standard deviation of double determinations).**

| LAB cell counts [Log cfu/ml] | | Folate [ng/ml] |
|---|---|---|
| 0 h | d3 - d7^{a,b} | d3-d7^{a,c} |
| 5.1 | 9.62 | 4293 |
| ± 0.35 | ± 0.01 | ± 193 |

| | | |
|---|---|---|
| a) average values over several days were calculated for constant cell growth / vitamin production to show the stability. b) average daily cell counts from day 3 to 7. c) average daily folate concentrations from day 3 to 7. | | |

### References

Bekaert, S., S. Storozhenko, P. Mershah, M.J. Bennett, W. Lambert, J.F. Gregory, K. Schubert, J. Hugenholtz, D. Van der Straeten, and A.D. Hanson. 2008. Folate bilofortification in food plants. Trends in Plant Science. 13:28-35.
Berry, E.C., and L.B. Bullerman. 1966. Use of cheese whey for vitamin B12 production. II. cobalt precursor and aeration levels. Applied Microbiology. 3:356-357.
Cerna, J. and Hrabova, H. 1977. Biological enrichment of fermented milk beverages with vitamin B12 and folic acid. Milchwissenschaft, 32: 274-277.
De Man, J.D., M. Rogosa, and M.E. Sharpe. 1960. A medium for the cultivation of lactobacilli. Journal of Applied Bacteriology. 23:130-135.
Eurolex. 2003. Amtsblatt Nr. L 006 vom 10/01/2003 S. 0001 - 0089.
Forssén, K.M., M.I. Jagerstad, K. Wigektz, and C.M. Witthoft. 2000. Folates and dairy products: A critical update. Journal of the American College of Nutrition. 19:100S-110S.
Frank, J. 2002. Vitamin B12, p. 75-79. In Biesalski, H.K., J. Köhrle, and K. Schümann, Vitamine, Spurenelemente und Mineralstoffe. Georg Thieme Verlag, Germany.
Galkina, G.V., Illarionova, V.L., Kuksova, E.V., Gorbatova, E.V. and Volkova, G.S., 2003. Method for preparing protein-vitamin fodder. RU2243678.
Gonzales Siso, M.I. 1996. The biotechnological utilization of cheese whey: A review. Bioresource Technology. 57:1-11.
Grinstead, D.A., and S.F. Barefoot. 1992. Jenseniin G, a heat-stable bacteriocin produced by Propionibacterium jensenii P126. Applied and Environmental Microbiology. 58:215-220.
Hettinga, D.H., and G.W. Reinbold. 1972. Propionic-Acid Bacteria - Review .1. Growth. Journal of Milk and Food Technology. 35:295-301.
Horne, D.W., and D. Patterson. 1988. Lactobacillus casei microbiological assay of folic acid derivatives in 96-well microtiter plates. Clinical Chemistry. 34:2357-2359.
Hugenholtz, J., J. Hunik, H Santos, and E. Smid. 2002. Nutraceutical production by propionibacteria. Lait. 82:103-112.
Hugenholtz, J., and E.J. Smid. 2002. Nutraceutical production with food-grade microorganisms. Current Opinion in Biotechnology. 13:497-507.
Johnson, M.A. 2007. If high folic acid aggravates Vitamin B-12 deficiency what should be done about it? Nutrition Reviews. 65:451-458.
Leroy, F., and L. De Vuyst. 2004. Lactic acid bacteria as functional starter cultures for the food fermentation industry. Trends in Food Science & Technology. 15: 67-78.
Liu, J.A.P., and N.J. Moon. 1982. Commensalistic interaction between Lactobacillus-acidophilus and Propionibacterium-shermanii. Applied and Environmental Microbiology. 44:715-722.
Lucock, M. 2000. Folic Acid: Nutritional biochemistry, molecular biology, and role in disease processes. Molecular Genetics and Metabolism. 71:121-138.
Lund, B., B. Norddahi, and B. Ahring. 1992. Production of lactic-acid from whey using hydrolyzed whey-protein as nitrogen-source. 14:851-856.
Martens, J.H., H. Barg, M.J. Warren, and D. Jahn. 2002. Microbial production of vitamin B-12. Applied Microbiology and Biotechnology. 58:275-285.
Marwaha, S.S., R.P. Sethi, and J.F. Kennedy. 1983. Influence of 5,6-dimethylbenzimidazole (DMB) on vitamin B12 biosynthesis by strains of Propionibacterium. Enzyme and Microbial Technology. 5:361-364.
Mawson, J. 2003. Whey and whey powders / Fermentation of Whey, p. 6157-6163. In Mawson, J., and B. Caballero, Encyclopedia of food sciences and nutrition. Academic press, Oxford.
Miescher Schwenninger, S., C. Lacroix, S. Truttmann, C. Jans, C. Spörndli, L. Bigler, and L. Meile. 2008. Characterization of low-molecular weight antiyeast metabolites produced by a food-protective Lactobacillus/Propionibacterium co-culture. Journal of Food Protection. 71:2481-2487.
Miescher Schwenninger, S., and L. Meile. 2004. A mixed culture of Propionibacterium jensenii and Lactobacillus paracasei subsp. paracasei inhibits food spoilage yeasts. Systematic and Applied Microbiology. 27:229-237.
Murooka, Y., Y. Piao, P. Kiatpapan, and M. Yamashita. 2005. Production of tetrapyrrole compounds and vitamin B-12 using genetically engineering of Propionibacterium freudenreichii. An overview. Lait. 85:9-22.
Panesar, P.S., J.F. Kennedy, D.N. Gandhi, and K. Bunko. 2007. Bioutilisation of whey for lactic acid production. Food Chemistry. 105:1-14.
Piao, Y., M. Yamashita, N. Kawaraichi, R. Asegawa, H. Ono, and Y., Murooka. 2004. Production of vitamin B-12 in genetically engineered Propionibacterium freudenreichii. Journal of Bioscience and Bioengineering. 98:167-173.
Quinlivan, E.P., A.D. Hanson, and J.F. Gregory. 2006. The analysis of folate and its metabolic precursors in biological samples. Analytical Biochemistry. 348:163-184.
Refsum, H., and A.D. Smith. 2008. Are we ready for mandatory fortification with vitamin B-12? American Journal of Clinical Nutrition. 88:253-254.
Reynolds, E. 2006. Vitamin B12, folic acid, and the nervous system. Lancet Neurology. 5:949-960.
Ryan-Harshmann, M., and W. Aldoori. 2008. Vitamin B12 and health. Canadian Family Physician. 54:536-541.
Sieuwerts, S., F.A.M. de Bok, J. Hugenholtz, and J. Vlieg. 2008. Unraveling microbial interactions in food fermentations: from classical to genomics approaches. Applied and Environmental Microbiology. 74:4997-5007.
Stover, P.J. 2004. Physiology of folate and vitamin B-12 in health and disease. Nutrition Reviews. 62:S3-S12.
Suomalainen, T.H., and A.M. Mäyrä-Mäkinen. 1999. Propionic acid bacteria as protective cultures in fermented milks and breads. Lait. 79:165-174.
Survase, S.A., I.B. Bajaj, and R.S. Singhai. 2006. Biotechnological production of vitamins. Food Technology and Biotechnology. 44:381-396.
Sybesma, W., M. Starrenburg, L. Tijsseling, M.H.N. Hoefnagel, and J. Hugenholtz. 2003. Effects of cultivation conditions on folate production by lactic acid bacteria. Applied and Environmental Microbiology. 69:4542-4548.
Tönz, O. 2002. Vom Sinn und Zweck einer generellen Folsäure-Prophylaxe. Forum Med Suisse. 27:303-31.
Truswell, A.S. 2007. Vitamin B12. Nutrition and Dietetics. 64:S120-S125.
Ulrich, C.M., and J.D. Potter. 2006. Folate supplementation: Too much of a good thing? Cancer Epidemiology Biomarkers and Prevention. 15:189-193.
Vasala, A., J. Panula, and P. Neubauer. 2005. Efficient lactic acid production from high salt containing dairy by-products by Lactobacillus salivarius ssp. salicinius with pre-treatment by proteolytic microorganisms. Journal of Biotechnology. 117:421-431.
Wehrli, C. 1995. Method for the production of folic acid. United Stated Patent 5410056. 1-8.
Winkels, R.M., I.A. Brouwer, R.Clarke, M.B. Katan, and P. Verhoef. 2008. Bread cofortified with folic acid and vitamin B-12 improves the folate and vitamin B-12 status of healthy older people: a randomized controlled trial. American Journal of Clinical Nutrition. 88:348-355.
Witthöft and Jägerstad, 2002. VITAMINS / Folates, Nutritional Significance, p. 2714-2721. In Witthöft, C.M., M. Jagerstad, and H. Roginski, Encyclopedia of dairy sciences. Elsevier, Oxford.
Wright, A.J.A., J.R. Dainty, and P.M. Finglas. 2007. Folic acid metabolism in human subjects revisited: potential implications for proposed mandatory folic acid fortification in the UK. British Journal of Nutrition. 98:667-675.

## Claims

1. A process for the preparation of a fermentation broth, **characterized in that** a mixture of
- *Lactobacillus plantarum* SM39,
- *Propionibacterium freudenreichii* DF13 and
- supplemented whey permeate, whereby said whey permeate is preferably obtained either from ultrafiltration of milk or whey, whereby said whey is a by-product from cheese production, and whereby said supplemented whey permeate contains added cobalt chloride in an amount of 5 mg/l,
whereby said supplementation comprises the addition of
- 0.02 % (w/v) magnesium sulfate,
- 0.005 % (w/v) manganese sulfate,
- 1 % (w/v) yeast extract,
- p-amino benzoic acid in an amount of 10 mg/l,
- 5,6-dimethylbenzimidazole in an amount of 15 mg/l and
- a buffer consisting of a 0.1 M aqueous solution of K₂HPO₄ and KH₂PO₄ having a pH value of 6.4,
whereby 10⁵ cfu/ml of said *Lactobacillus plantarum* SM39 and 10⁷ cfu/ml of said *Propionibacterium freudenreichii* DF13 are present at the beginning of the cultivation,
is prepared, then
this mixture is cultivated for 72 hours under anaerobic conditions under a N₂ atmosphere, followed by a cultivation under aerobic conditions during at least 24 hours, preferably during 72 hours,
whereby said cultivation is carried under stirring at a temperature of 33°C at a pH value of 6.1 and whereby said pH value is controlled by the addition of 5 M NaOH, and
finally the resulting fermentation broth is collected.

2. Use of a fermentation broth prepared according to the process of claim 1
-- as an additive for food, or for a food supplement, or
-- for the preparation of a medicament for the prevention and/or treatment of vitamin deficiencies.

3. Use according to claim 2, **characterized in that** said food is a bakery product, especially a bread, or a beverage, preferably a beverage containing also aroma components.

4. A process for the preparation of a fermentation product, **characterized in that**
- a fermentation broth according to the process of claim 1 is prepared,
- from this fermentation broth the lactic acid bacteria, the propionic acid bacteria, the folate present in intracellular form in said lactic acid bacteria, and the vitamin B12 present in intracellular form in said propionic acid bacteria, are separated into a first liquid phase and a bacteria containing phase,
- the so separated bacteria containing phase is subjected to a cell disruption process,
- the disrupted product is subjected to a separation step, whereby the bacterial cell debris are removed, and
- the so obtained second liquid phase, containing the deliberated folate and vitamin B12, and the first liquid phase are combined.

5. The process according to claim 4, **characterized in that** said separation steps are realized by centrifugation or filtration, especially a filtration with a pore size of 0.45 µm or less.

6. The process according to one of claims 4 to 5, **characterized in that** the cell disruption process comprises either
- a heat treatment, especially a heating to a temperature in the range from 95°C to 150°C during a time from 2 seconds to 15 minutes, or
- a shearing treatment, especially a shearing treatment with quartz sand or with a "French Press" or a homogenization.

7. The process according to one of claims 4 to 6, **characterized in that** the fermentation product is dried, preferably under vacuum, especially by using the vacuum belt technology, using a temperature in a range from 35°C to 45°C and gum arabicum, maltodextrine, or cyclodextrine as carrier material in an amount of 5 to 70 % (w/w).

8. Use of the fermentation product, prepared according to one of claims 4 to 7
- as a food, or
- as an additive for food, or
- for the preparation of a medicament for the prevention and/or treatment of vitamin deficiencies.

9. Use according to claim 8, **characterized in that** said food is a bakery product, especially a bread, or a beverage, preferably a beverage containing also aroma components.

## Patentansprüche

1. Verfahren zur Herstellung einer Fermentationsbrühe, **dadurch gekennzeichnet, dass** ein Gemisch von
- *Lactobacillus plantarum* SM39,
- *Propionibacterium freudenreichii* DF13 und
- angereichertes Molken-Permeat, wobei das genannte Molken-Permeat vorzugsweise erhalten wird durch Ultrafiltration von Milch oder Molke, wobei die genannte Molke ein Nebenprodukt aus der Käseherstellung ist, und wobei das genannte angereicherte Molken-Permeat hinzugefügtes Kobaltchlorid in einer Menge von 5 mg/l enthält,
wobei die genannte Anreicherung die Hinzugabe von
- 0,02 % (w/v) Magnesiumsulfat,
- 0,005 % (w/v) Mangansulfat,
- 1 % (w/v) Hefeextrakt,
- p-Aminobenzoesäure in einer Menge von 10 mg/l,
- 5,6-Dimethylbenzimidazol in einer Menge von 15 mg/l und
- einem Puffer, bestehend aus einer 0,1 M wässrigen Lösung von K₂HPO₄ und KH₂PO₄ mit einem pH Wert von 6,4,
umfasst, wobei 10⁵ KBE/ml an genanntem Lactoba*cillus plantarum* SM39 und 10⁷ KBE/ml an genanntem Propio*nibacterium freudenreichii* DF13 am Beginn der Kultivierung vorhanden sind,
hergestellt wird, dann
dieses Gemisch während 72 Stunden unter anaeroben Bedingungen unter einer N₂ Atmosphäre kultiviert wird, gefolgt von einer Kultivierung unter aeroben Bedingungen während wenigstens 24 Stunden, vorzugsweise während 72 Stunden,
wobei die genannte Kultivierung unter Rühren bei einer Temperatur von 33°C bei einem pH Wert von 6,1 ausgeführt wird, und wobei der genannte pH Wert durch die Hinzugabe von 5 M NaOH kontrolliert wird, und
schlussendlich die resultierende Fermentationsbrühe gesammelt wird.

2. Verwendung einer Fermentationsbrühe, hergestellt gemäss dem Verfahren nach Anspruch 1,
- als Zusatzstoff in einem Nahrungsmittel oder einem Nahrungsergänzungsmittel, oder
- für die Herstellung eines Medikamentes für die Vorbeugung und/oder Behandlung von Vitaminmängeln.

3. Verwendung nach Anspruch 2, **dadurch gekennzeichnet, dass** das genannte Nahrungsmittel ein Bäckereiprodukt, insbesondere ein Brot, oder ein Getränk ist, vorzugsweise ein Getränk, welches auch Aromakomponenten enthält.

4. Verfahren zur Herstellung eines Fermentationsproduktes, **dadurch gekennzeichnet, dass**
- eine Fermentationsbrühe gemäss dem Verfahren nach Anspruch 1 hergestellt wird,
- aus dieser Fermentationsbrühe die Milchsäurebakterien, die Propionsäurebakterien, das Folat, welches in intrazellulärer Form in den genannten Milchsäurebakterien vorhanden ist, und das Vitamin B12, welches in intrazellulärer Form in den genannten Propionsäurebakterien vorhanden ist, in eine erste flüssige Phase und eine Bakterien enthaltende Phase getrennt werden,
- die so abgetrennte, Bakterien enthaltende Phase einem Zellspaltungsverfahren unterworfen wird,
- das gespaltene Produkt einem Abtrennungsschritt unterworfen wird, wobei die bakteriellen Zellbruchstücke entfernt werden, und
- die so erhaltene zweite flüssige Phase, welche das freigesetzte Folat und das Vitamin B12 enthält, und die erste flüssige Phase vereint werden.

5. Verfahren nach Anspruch 4, **dadurch gekennzeichnet, dass** die genannten Trennschritte durch Zentrifugation oder Filtration realisiert werden, insbesondere eine Filtration mit einer Porengrösse von 0,45 µm oder weniger.

6. Verfahren nach einem der Ansprüche 4 bis 5, **dadurch gekennzeichnet, dass** das Zellspaltungsverfahren entweder
- eine Wärmebehandlung, insbesondere eine Erwärmung auf eine Temperatur in Bereich von 95°C bis 150°C während 2 Sekunden bis 15 Minuten, oder
- eine Zerschneid-Behandlung, insbesondere eine Zerschneid-Behandlung mit Quarzsand oder mit einer "French Press" oder eine Homogenisierung,
umfasst.

7. Verfahren nach einem der Ansprüche 4 bis 6, **dadurch gekennzeichnet, dass** das Fermentationsprodukt getrocknet wird, vorzugsweise unter Vakuum, insbesondere unter Verwendung der Saugband-Technologie, unter Verwendung einer Temperatur im Bereich von 35°C bis 45°C und Gummi arabicum, Maltodextrin oder Cyclodextrin als Trägermaterial in einer Menge von 5 bis 70 % (w/w).

8. Verwendung des Fermentationsprodukt, hergestellt nach einem der Ansprüche 4 bis 7,
- als Nahrungsmittel, oder
- als Zusatzstoff in einem Nahrungsmittel, oder
- für die Herstellung eines Medikamentes für die Vorbeugung und/oder Behandlung von Vitaminmängeln.

9. Verwendung nach Anspruch 8, **dadurch gekennzeichnet, dass** das genannte Nahrungsmittel ein Bäckereiprodukt, insbesondere ein Brot, oder ein Getränk ist, vorzugsweise ein Getränk, welches auch Aromakomponenten enthält.

## Revendications

1. Procédé pour la préparation d'un moût de fermentation, **caractérisé en ce qu'**un mélange de
- *Lactobacillus plantarum* SM39,
- *Propionibacterium freudenreichii* DF13 et
- perméat de lactosérum supplémenté, par quoi ledit perméat de lactosérum est de préférence obtenu à partir de l'ultrafiltration de lait ou de lactosérum, par quoi ledit lactosérum est un sous-produit de la production de fromage, et par quoi ledit perméat de lactosérum supplémenté contient du chlorure de cobalt ajouté dans une quantité de 5 mg/l,
par quoi ladite supplémentation comprend l'addition de
- 0,02% (p/v) de sulfate de magnésium,
- 0,005% (p/v) de sulfate de manganèse,
- 1% (p/v) d'extrait de levure,
- acide p-aminobenzoïque dans une quantité de 10 mg/l,
- 5,6-diméthylbenzimidazole dans une quantité de 15 mg/l et
- un tampon constitué d'une solution aqueuse 0,1 M de K₂HPO₄ et KH₂PO₄ ayant une valeur de pH de 6,4,
par quoi 10⁵ cfu/ml dudit *Lactobacillus plantarum* SM39 et 10⁷ cfu/ml dudit *Propionibacterium freudenreichii* DF13 sont présentes au début de la culture,
est préparé, ensuite
ce mélange est cultivé pendant 72 heures sous des conditions anaérobies sous une atmosphère de N₂, suivies par une culture sous des conditions aérobies durant au moins 24 heures, de préférence durant 72 heures,
par quoi ladite culture est effectuée sous agitation à une température de 33°C à une valeur de pH de 6,1 et par quoi ladite valeur de pH est contrôlée par l'addition de 5 M NaOH, et
finalement le moût de fermentation résultant est récolté.

2. Utilisation d'un moût de fermentation préparé selon le procédé de la revendication 1
en tant qu'additif pour aliments ou pour un supplément alimentaire,
ou pour la préparation d'un médicament pour la prévention et/ou le traitement de déficiences en vitamines.

3. Utilisation selon la revendication 2 **caractérisée en ce que** ledit aliment est un produit de boulangerie, spécialement un pain, ou une boisson, de préférence une boisson contenant aussi des composants aromatiques.

4. Procédé pour la préparation d'un produit de fermentation, **caractérisé en ce que**
- un moût de fermentation selon le procédé de la revendication 1 est préparé,
- à partir de ce moût de fermentation, les bactéries produisant de l'acide lactique, les bactéries produisant de l'acide propionique, le folate présent sous la forme intracellulaire dans lesdites bactéries produisant de l'acide lactique, et la vitamine B12 présente sous la forme intracellulaire dans lesdites bactéries produisant de l'acide propionique, sont séparés en une première phase liquide et une phase contenant les bactéries,
- la phase ainsi séparée contenant les bactéries est soumise à un procédé de désintégration des cellules,
- le produit désintégré est soumis à une étape de séparation, par laquelle les débris cellulaires des bactéries sont éliminés, et
- la seconde phase liquide ainsi obtenue, contenant le folate et la vitamine B12 libérés, et la première phase liquide sont combinées.

5. Procédé selon la revendication 4, **caractérisé en ce que** lesdites étapes de séparation sont réalisées par centrifugation ou filtration, en particulier une filtration avec une taille de pore de 0,45 µm ou moins.

6. Procédé selon l'une des revendications 4 à 5, **caractérisé en ce que** le procédé de désintégration des cellules comprend soit
- un traitement à la chaleur, en particulier un chauffage à une température dans la plage de 95°C à 150°C durant un temps de 2 secondes à 15 minutes, soit
- un traitement de cisaillement, en particulier un traitement de cisaillement avec du sable de quartz ou avec une « presse de French » ou une homogénéisation.

7. Procédé selon l'une quelconque des revendications 4 à 6, **caractérisé en ce que** le produit de fermentation est séché, de préférence sous vide, en particulier à l'aide de la technologie de bande sous vide, en utilisant une température dans une plage de 35°C à 45°C et de la gomme arabique, de la maltodextrine, ou de la cyclodextrine en tant que matériau support dans une quantité de 5 à 70% (p/p).

8. Utilisation du produit de fermentation, préparé selon l'une quelconque des revendications 4 à 7
- en tant qu'aliment, ou
- en tant qu'additif pour aliment, ou
- pour la préparation d'un médicament pour la prévention et/ou le traitement de déficiences en vitamines.

9. Utilisation selon la revendication 8, **caractérisé en ce que** ledit aliment est un produit de boulangerie, en particulier un pain, ou une boisson, de préférence une boisson contenant aussi des composants aromatiques.
